# EUROPÄISCHE PATENTSCHRIFT

(11) **EP 3 478 173 B1**
(45) Veröffentlichungstag und Bekanntmachung des Hinweises auf die Patenterteilung: **09.09.2020**
(21) Anmeldenummer: 17730037.3
(22) Anmeldetag: 31.05.2017
(51) Int. Cl.: A61B 5/11

(54) **VERFAHREN ZUR BESTIMMUNG DER FÜR EINEN TIMED-UP-AND-GO-TEST BENÖTIGTEN ZEIT**
METHOD FOR DETERMINING THE TIME REQUIRED FOR A TIMED UP AND GO TEST
PROCÉDÉ POUR DÉTERMINER LE TEMPS REQUIS POUR UN TEST DE LOCOMOTION ET D'ÉQUILIBRE CHRONOMÉTRÉ

(30) Priorität: 30.06.2016 AT 505902016
(43) Veröffentlichungstag der Anmeldung: 08.05.2019
(73) Patentinhaber: AIT Austrian Institute of Technology GmbH, 1220 Wien (AT)
(72) Erfinder: MODRE-OSPRIAN, Robert, 8563 Ligist (AT); KASTNER, Peter, 8054 Seiersberg-Pirker (AT); SCHREIER, Günter, 8010 Graz (AT); SÁNCHEZ SÁNCHEZ, Alberto, 28903 Getafe (Madrid) (ES)
(74) Vertreter: Wildhack & Jellinek
(86) Internationale Anmeldenummer: PCT/AT2017/060140
(87) Internationale Veröffentlichungsnummer: WO 2018/000007

(56) Entgegenhaltungen:
- JP-A- 2015 216 953
- FRENKEN T ET AL: "aTUG: Fully-automated timed up and go assessment using ambient sensor technologies", 2011 5TH INTERNATIONAL CONFERENCE ON PERVASIVE COMPUTING TECHNOLOGIES FOR HEALTHCARE (PERVASIVEHEALTH 2011) IEEE PISCATAWAY, NJ, USA, 2011, Seiten 55-62, XP002772748, ISBN: 978-1-61284-767-2
- GONCALVES J ET AL: "Fully-automated strength, agility and endurance tests assessment: An integrated low cost approach based on an instrumented chair", PROCEEDINGS OF THE 2014 IEEE EMERGING TECHNOLOGY AND FACTORY AUTOMATION (ETFA) IEEE PISCATAWAY, NJ, USA, 2014, Seite 6 pp., XP002772749, ISBN: 978-1-4799-4845-1

## Beschreibung

Die Erfindung betrifft ein Verfahren zur Bestimmung der Zeit, die eine Person für die Durchführung eines Timed-Up-and-Go-Tests benötigt. Weiters betrifft die Erfindung eine Vorrichtung, mit dem ein Timed-Up-and-Go-Test durchgeführt werden kann.

Bei einem Timed-Up-and-Go-Test handelt es sich um einen Test, bei dem ein Proband auf einem Stuhl sitzt. In einer möglichen Ausführungsform wird der Stuhl in einer vorgegebenen Entfernung von vorzugsweise drei Metern von einer Wand oder einem anderen Hindernis aufgestellt. Alternativ kann der Stuhl auch in einer solchen Entfernung von einem Hindernis oder einer Bodenmarkierung aufgestellt sein, um dem Probanden anzuzeigen, an welcher Steller er später umkehren soll. Schließlich besteht auch die Möglichkeit, die Position der Umkehr für den Probanden völlig offen zu lassen.

Der Proband wird angewiesen, vom Stuhl aufzustehen, zur Wand zu gehen, im Bereich der Wand, des Hindernisses, der Markierung oder in einem bestimmten Abstand vom Stuhl umzukehren, zum Stuhl zurückzukehren und sich wieder auf den Stuhl hinzusetzen. Die für die Durchführung dieser Aufgabe gemessene Zeit wird festgehalten. Die Zeit, die der Proband für die Durchführung der Aufgabe benötigt, gibt Aufschluss über die motorischen Fähigkeiten des Probanden.

Aus dem Stand der Technik ist sowohl der Timed-Up-and-Go-Test bekannt wie auch unterschiedliche Möglichkeiten von mechanischen Hilfsmitteln zur Durchführung des Timed-Up-and-Go-Tests. Dokument JP 2015 216953 A beschreibt einen Timed-Up-and-Go-Test. Bei all aus dem Stand der Technik bekannten Verfahren werden Mittel eingesetzt, die die Position des Probanden in Bezug auf die Testanordnung bestimmen. Die Durchführung des Tests mit automatisierten Anordnungen ist jedoch relativ fehleranfällig, sodass es im Moment nicht möglich ist, eine Anordnung zu erreichen, die mit einfachen Mitteln leicht erstellbar und konfigurierbar ist. Die Erfindung stellt sich die Aufgabe, die vorstehend genannten Probleme zu lösen und ein Verfahren zur Durchführung eines Timed-Up-and-Go-Tests zur Verfügung zu stellen, das einfach durchzuführen ist, und zwar nahezu unabhängig von den zur Verfügung stehenden räumlichen Gegebenheiten.

Die Erfindung löst diese Aufgabe mit einem Verfahren zur Bestimmung der für einen Timed-Up-and-Go-Test benötigten Zeit gemäß dem Patentanspruch 1.

Dabei ist vorgesehen, dass zur Bestimmung der für einen Timed-Up-and-Go-Test benötigten Zeit sowie zur Verifikation des ermittelten Ergebnisses,
- wobei ein Entfernungsmessgerät vorgesehen ist, das den Abstand eines vor einem Stuhl befindlichen Probanden von der Rückenlehne des Stuhls laufend misst, und an eine Steuereinheit weiterleitet,
- wobei der Stuhl positioniert wird, insbesondere indem er zu einer Wand oder einem Hindernis oder einer Markierung in einen vorgegebenen Abstand gebracht wird,
- wobei die vom Entfernungsmessgerät aufgezeichneten Entfernungsmesswerte (d) laufend aufgezeichnet werden,
- wobei der Proband angewiesen wird, vom Stuhl aufzustehen, vorwärts, insbesondere zur Wand, zum Hindernis oder zur Markierung zu gehen, anschließend, insbesondere vor der Wand oder dem Hindernis oder im Bereich der Markierung, umzukehren und sich wieder auf dem Stuhl hinzusetzen und der Proband diese Anweisung nach seinen Möglichkeiten ausführt,
- wobei überprüft wird, ob der Proband auf dem Stuhl sitzt und wenn dies für eine vorgegebene Zeitspanne der Fall ist, eine Zeitnehmung gestartet wird und ein vorgegebenes Start-Signal abgegeben wird,
- wobei die Zeitnehmung gestoppt wird, wenn sich der Proband wieder auf den Stuhl setzt,
- wobei aus den über die Zeit aufgenommenen Entfernungsmesswerten ein Signal (d(t)) erstellt wird, das insbesondere nach dem Ende der Zeitnehmung, daraufhin untersucht wird, ob es
   a) in einem ersten Zeitbereich kontinuierlich ansteigt, gegebenenfalls auch für einzelne Zeiträume bis auf Messungenauigkeiten unverändert bleibt, und
   b) nach dem Übersteigen eines vorgegebenen Umkehrschwellenwerts (d_{th,u}) wieder kontinuierlich abnimmt, gegebenenfalls auch für einzelne Zeiträume bis auf Messungenauigkeiten unverändert bleibt, bis es den unteren Schwellenwert (d_{th,s}) unterschreitet, und
- sofern die Vorraussetzungen a) und b) vorliegen, der Test als korrekt durchgeführt angesehen wird und die hierfür benötigte Zeitdauer der Zeitnehmung zur Verfügung gehalten, gegebenenfalls, angezeigt wird.

Mit diesem Verfahren ist es auf einfache Weise möglich, festzustellen ob der Timed-Up-and-Go-Test korrekt durchgeführt wurde und die hierfür benötigte Zeitdauer einfach zu erhalten. Insbesondere kann dieser Test vom Probanden selbst ohne Hilfe durch eine weitere Person durchgeführt werden.

Eine besonders bevorzugte Durchführung des Tests, die einen automatischen Start erlaubt, sieht vor, dass vor der Ausführung des Tests die ermittelten Entfernungsmesswerte laufend untersucht werden und für den Fall, dass die Entfernungsmesswerte für eine vorgegebene Zeitspanne einen unteren Schwellenwert unterschreiten, insbesondere wenn der Proband sitzt, das Start-Signal abgegeben wird, das die Zeitnehmung auslöst dem Probanden signalisiert, dass der Test beginnt.

Um eine Testausführung durch eine weitere Person unabhängig vom Probanden durchführen zu können, kann vorgesehen sein, dass ein externes Triggersignal die Abgabe des Start-Signals und den Beginn der Zeitnehmung veranlasst.

Alternativ kann zu diesem Zweck auf vorgesehen sein, dass die Zeitnehmung gestartet wird, wenn ein in der Sitzfläche des Stuhls gelegener Sensor, insbesondere Drucksensor den Abfall des Drucks unter einen vorgegebenen Schwellenwert feststellt.

Zur automatischen Beendigung eines Tests kann vorgesehen sein, dass , für den Fall, dass die Entfernungsmesswerte nach erstmaliger und einen vorgegebenen Zeitraum andauernder Überschreitung eines weiteren Schwellenwerts für einen vorgegebene Zeitspanne einen unteren Schwellenwert unterschreitet, insbesondere wenn der Proband (1) wieder auf dem Stuhl (4) sitzt, der Test beendet und die Zeitnehmung gestoppt wird.

Zur Beendigung des Tests durch eine Person kann vorgesehen sein, dass ein externes Triggersignal die Zeitnehmung beendet, oder dass die Zeitnehmung beendet wird, wenn ein in der Sitzfläche des Stuhls gelegener Sensor, insbesondere Drucksensor, einen Schwellenwert übersteigenden Druck feststellt.

Eine einfache Verifikation bzw. Falsifikation der Testergebnisse sieht vor, dass der Test als inkorrekt angesehen wird,
- wenn die Steigung des Signals (d(t)) einen vorgegebenen Steigungsschwellenwert überschreitet und/oder
- wenn das Signal (d(t)) Messwerte aufweist, die der Entfernung des Stuhls (5) von der Wand (2) oder vom Hindernis entsprechen oder Messwerte aufweist, die die Entfernung des Stuhls zur Markierung um einen vorgegebenen Schwellenwert übersteigen, und/oder
- wenn keiner der Entfernungsmesswerte (d) des Signals (d(t)) einen vorgegebenen Schwellenwert (d_{th,u}) erreicht und der Proband den Umkehrbereich (32) nicht erreicht, und/oder
- wenn die Zeitnehmung eine Maximalzeit erreicht, ohne dass der Test beendet wird.

Eine Prüfung, ob das Entfernungsmessgerät während der gesamten Messung gegenüber der Wand korrekt ausgerichtet ist, sieht vor, dass die Ausrichtung des Entfernungsmessgeräts gegenüber der Wand oder dem Hindernis laufend überwacht wird und bei Änderungen der Ausrichtung Entfernungsmessgeräts oder bei einen Schwellenwert übersteigenden Abweichungen der Ausrichtung des Entfernungsmessgeräts von einer vorgegebenen, insbesondere horizontalen und/oder normal zur Wand oder zum Hindernis stehenden, Ausrichtung eine Fehlermeldung ausgegeben und das Messergebnis invalid wird.

Eine einfache Erstellung eines Aufnahmesettings sieht vor, dass vor der Ausführung des Tests das Entfernungsmessgerät auf der Lehne des Stuhls angeordnet und von der Lehne in Richtung zum sitzenden Probanden hin ausgerichtet wird.

Weiters kann zur Initialisierung bzw. Erstellung der Ausgangsanordnung des Messsettings vorgesehen sein, dass die vor dem Beginn des Tests ermittelten Entfernungsmesswerte mit einem vorgegebenen Schwellenwert verglichen werden und derart eine korrekte Position des Stuhls festgestellt wird,
- wenn die ermittelten Entfernungsmesswerte innerhalb eines vorgegebenen Entfernungsbereichs liegen und/oder
- wenn die Ausrichtung des Entfernungsmessgeräts einer vorgegebenen, insbesondere horizontalen und/oder normal zur Wand oder zum Hindernis stehenden Ausrichtung entspricht.

Zur korrekten Positionierung des Stuhls gegenüber der Wand kann dabei bevorzugt vorgesehen sein, dass der Proband angewiesen wird, nach Beendigung des Tests aufstehen und hinter den Stuhl gehen oder den Aufnahmebereich des Entfernungsmessgeräts zu verlassen, und dass anschließend die erneut korrekte Position und/oder Ausrichtung des Stuhls erneut nach vorgegebenen Kriterien geprüft wird und der Test als inkorrekt angesehen wird, wenn die Position und/oder Ausrichtung des Stuhls zur Wand nicht diesen Kriterien entspricht.

Eine bevorzugte Entfernungsmessung sieht vor, dass das Entfernungsmessgerät die Entfernung mittels Ultraschall- oder Lasermessung bestimmt.Hierdurch kann eine weitere Erhöhung der Zuverlässigkeit des Messverfahrens erreicht werden.

Auf eine Markierung oder eine Wand oder ein Hindernis kann insbesondere dann verzichtet werden, wenn zur Veranlassung des Probanden zur Umkehr ein insbesondere akustisches Umkehrsignal abgegeben wird, wenn die Entfernungsmesswerte des Entfernungsmessgeräts einen vorgegebenen Abstand überschreiten, insbesondere dann, wenn sich der Proband im Umkehrbereich befindet.

Eine bevorzugte Ausführungsform der Erfindung wird anhand der folgenden Zeichnungsfiguren näher dargestellt.

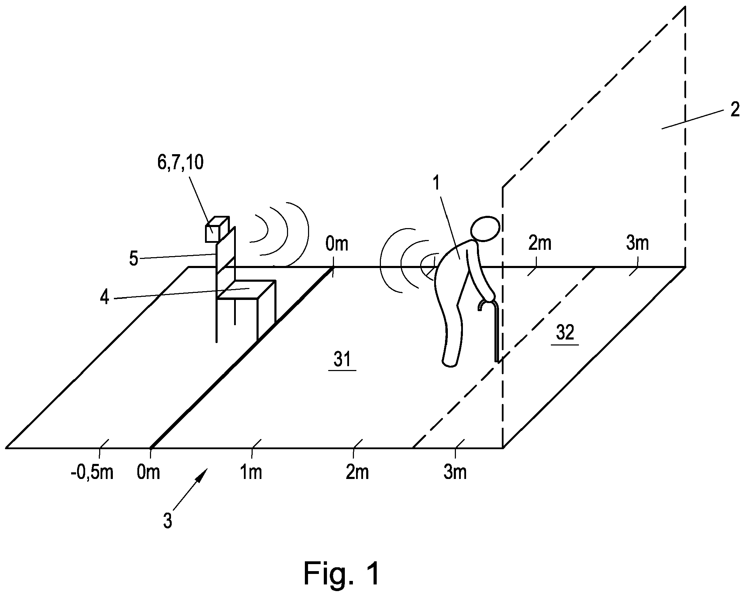

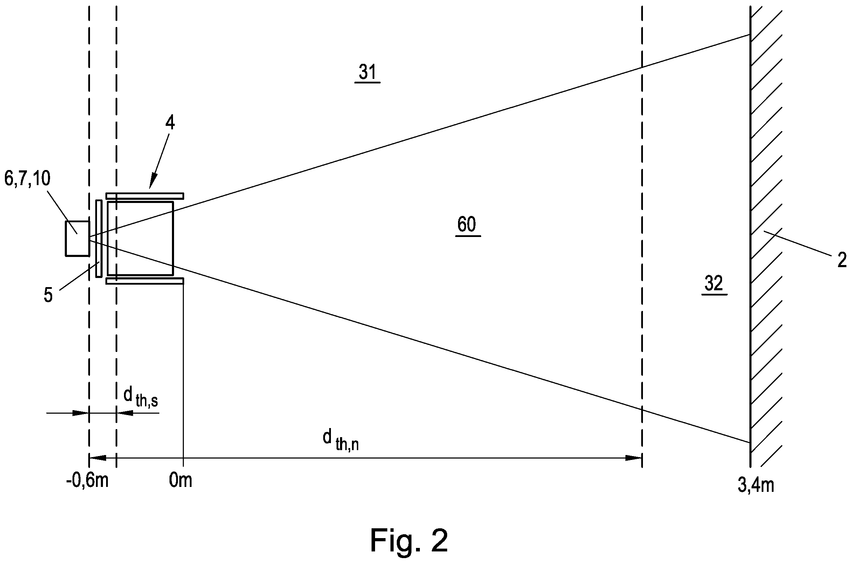

In **Fig. 1** ist eine einfache Anordnung zur Durchführung eines Timed-Up-and-Go-Tests dargestellt. **Fig. 2** zeigt die in **Fig. 1** dargestellte Anordnung schematisch von oben.

Zur Vorbereitung des Tests wird der Proband 1 in einen Testbereich **(****Fig.1****)** gebracht, in dem eine Wand 2 sowie vor der Wand 2 ein im Wesentlichen ebener Bodenbereich 3 zur Verfügung steht. Weiters steht im Bereich eine Vorrichtung umfassend einen Stuhl 4 mit einer Lehne 5 zur Verfügung. An der Lehne 5 wird vor der Ausführung des Tests ein Entfernungsmessgerät 6 angeordnet, das auf der Lehne 5 des Stuhls 4 angeordnet ist und von der Lehne 5 in Richtung eines auf dem Stuhl sitzenden Probanden 1 ausgerichtet ist. In einer Ausführungsvariante kann das Entfernungsmessgerät fest mit dem Stuhl 4 verbunden oder in den Stuhl 4, insbesondere in die Lehne 5 des Stuhls, integriert sein. In einem solchen Fall kann die konkrete Ausrichtung des Entfernungsmessgeräts in Bezug auf den Stuhl 4 entfallen. Auch die später genannte Ausrichtungsmessgerät 7 und Steuereinheit 10 können fest mit dem Stuhl 4 verbunden sein, insbesondere im Stuhl 4 oder in der Lehne 5 des Stuhls 4 integriert sein.

Der Stuhl 4 wird dabei so ausgerichtet, dass ein Proband 1, der in normaler Körperhaltung auf dem Stuhl 4 sitzt, frontal vor der Wand 2 sitzt. Bei dem Entfernungsmessgerät 6 handelt es sich um ein Ultraschall- oder LaserEntfernungsmessgerät, das in der in **Fig. 1** dargestellten Anordnung die Entfernung der Sessellehne 5 von der Wand 2 ermittelt.

Grundsätzlich ist es nicht erforderlich, dass im Testbereich eine massive Beton- oder Holzwand vorhanden ist. Es ist selbstverständlich auch möglich, dass es sich bei der für den Test genutzten Wand 2 um einen Schrank oder um eine mobile Trenn- oder Leinwand handelt. Es ist lediglich erforderlich, dass das Entfernungsmessgerät 6 die Wand 2 identifiziert, wenn sich im Zwischenbereich zwischen Entfernungsmessgerät 6 und Wand 2 keine Gegenstände oder vom Probanden 1 verschiedene Personen befinden.

Anstelle einer Wand 2 auch ein anderes Hindernis verwendet werden, das vom Entfernungsmessgerät 6 erfasst werden kann. Grundsätzlich ist es aber für die Durchführung des Tests nicht erforderlich, dass eine Wand 2 oder ein Hindernis verwendet wird. Es reicht vielmehr auch aus, wenn lediglich eine Markierung auf dem Boden ersichtlich ist, an der sich der Proband orientiert, um umzukehren.

Bei gut orientierten Probanden kann sogar auf eine solche Markierung verzichtet werden. Auch kann dann auf eine Markierung oder Wand verzichtet werden, wenn dem Probanden 1 ein Umkehr-Signal gegeben wird. Dieses kann entweder von einer externen, den Test begleitenden Person oder aufgrund der von dem Entfernungsmessgerät ermittelten Entfernungsmesswerten ermittelt werden, wenn diese einen vorgegebenen Schwellenwert überschreiten.

Befindet sich der Proband 1 nicht im Messbereich des Entfernungsmessgeräts 6, gibt das Entfernungsmessgerät 6 seinen Abstand von der Wand an. Zu diesem Zweck ist das Entfernungsmessgerät 6 horizontal und normal zur Wand 2 hin ausgerichtet. Gegebenenfalls kann ein Ausrichtungsmessgerät 7 vorgesehen sein, das feststellt, ob das Entfernungsmessgerät 6 horizontal ausgerichtet ist.

Darüber hinaus kann zusätzlich festgestellt werden, ob das Entfernungsmessgerät 6 normal zur Wand 2 hin ausgerichtet ist. Das Entfernungsmessgerät 6 und gegebenenfalls das Ausrichtungsmessgerät 7 sind an eine Steuereinheit 10 angeschlossen, die dem Entfernungsmessgerät 6 nachgeschaltet ist und der die einzelnen Entfernungsmesswerte d des Entfernungsmessgeräts 6 zugehen.

Während der Initialisierungsphase prüft die Steuereinheit 10 neben der korrekten Ausrichtung des Entfernungsmessgeräts 6 zusätzlich die Entfernungsmesswerte d, d.h. den Abstand der Lehne 5 von der Wand 2, und überprüft, ob die derart ermittelten Entfernungsmesswerte d innerhalb eines vorgegebenen Entfernungsbereichs, im vorliegenden Fall von etwa 3,5 Meter, liegen. Stellt die Steuereinheit 10 fest, dass die Entfernung der Lehne 5 des Stuhls 4 von der Wand 2 bezogen auf den für den Test erforderlichen Entfernungsbereich zu groß oder zu klein ist, wird dies auf einer auf der Steuereinheit 10 befindlichen Anzeigeeinheit dargestellt.

Die Erfindung ist jedoch nicht auf die hier vorgeschlagenen beispielhaften Abmessungen beschränkt. So ist es ohne weiteres möglich, die vom Probanden zurückzulegende Entfernung auch zu verlängern oder auch den Umkehrbereich zu vergrößern. Letzteres ist beispielsweise dann vorteilhaft, wenn der Proband nicht ohne Hilfsmittel gehen kann und die Gehhilfe, beispielsweise ein Rollator einen größeren Umkehrbereich, beispielsweise von einem Meter, erfordert. Für Probanden, die ohne Hilfsmittel gehen können, kann ein Umkehrbereich mit einer Länge von etwa 40 bis 50 cm gewählt werden.

Nach der Initialisierung der Messanordnung setzt sich der Proband 1 auf den Stuhl 4. Dabei wird die gemessene Entfernung des Probanden 1 laufend gemessen. Unterschreiten die gemessenen Entfernungsmesswerte d einen vorgegebenen Startschwellenwert d_{th,s} - falls der Proband 1 auf dem Stuhl 4 sitzt, entsprechen die Entfernungsmesswerte d einer Entfernung von weniger als 10cm, sodass der Startschwellenwert auf 10cm gesetzt werden kann - für eine vorgegebene Zeitspanne, beispielsweise 5 Sekunden, so wird angenommen, dass der Proband 1 auf dem Stuhl 4 sitzt. Die Steuereinheit 10 gibt ein Startsignal ab, das dem Probanden 1 signalisiert, dass der Test beginnt. Zudem wird eine Zeitnehmung ausgelöst bzw. gestartet. Alternativ dazu besteht die Möglichkeit, dass die Zeitnehmung von einer den Test begleitenden Person gestartet wird, die dem Probanden 1 auch signalisiert, dass der Test beginnt.

Schon vor der Testdurchführung wurde der Proband 1 angewiesen, vom Stuhl 4 aufzustehen, zur Wand 2 zu gehen, in einem Umkehrbereich 32 vor der Wand 2 umzukehren und sich wieder auf dem Stuhl 4 hinzusetzten. Während der Testdurchführung werden laufend Entfernungsmesswerte d ermittelt und über die Zeit ausgewertet, wobei ein Signal d(t) erstellt wird, dass die ermittelten Entfernungsmesswerte d über die Zeit t angibt. Die Zeitnehmung wird gestoppt wenn der Proband 1 wieder auf den Stuhl 4 sitzt. Dies kann dadurch erfolgen, dass die Entfernungsmesswerte nach erstmaliger und einen vorgegebenen Zeitraum andauernder Überschreitung eines Umkehrschwellenwerts d_{th,u} von beispielsweise 3 Metern, für eine vorgegebene Zeitspanne einen unteren Schwellenwert, beispielsweise den Startschwellenwert, unterschreitet, die Zeitnehmung gestoppt wird. Dies ist insbesondere dann der Fall, wenn der Proband 1 wieder auf dem Stuhl 4 sitzt. Der Test wird beendet, die Zeitnehmung wird gestoppt.

Alternativ kann der Test auch von einer den Test überwachenden Person beendet werden, wobei diese Person ein Triggersignal aussendet, das die Zeitnehmung beendet. Weiters kann das Triggersignal auch von einem anderen Sensor, beispielsweise einem in der Sitzfläche des Stuhls 4 integrierten Drucksensor erstellt werden, wenn der ermittelte Druck einen vorgegebenen Schwellenwert übersteigt.

In weiterer Folge wird das Signal d(t) der Entfernungsmesswerte d über die Zeit t, das während der Durchführung des Tests aufgenommen wurde, analysiert. Dabei wird untersucht, ob das erstellte Signal d(t) in einem ersten Zeitbereich kontinuierlich ansteigt, gegebenenfalls auch für einzelne Zeiträume bis auf Messungenauigkeiten unverändert bleibt und ob das Signal d(t) nach dem Übersteigen eines vorgegebenen Umkehrschwellenwerts, der etwas geringer ist als der Abstand der Lehne 5 von der Wand 2, im Timed-Up-and-Go-Test üblicherweise bei 3m liegt, wieder kontinuierlich abnimmt. Auf diese Weise kann festgestellt werden, ob der Proband den Umkehrbereich 32 betreten hat. Nach Verlassen des Umkehrbereichs 32 soll das Signal d(t) kontinuierlich abnehmen. Auch in diesem Fall kann toleriert werden, dass der Signalwert für einzelne Zeiträume bis auf Messungenauigkeiten unverändert bleibt, so lange das Signal d(t) letztlich den unteren Schwellenwert, der einer Entfernung von etwa 10cm von der Sessellehne 5 entspricht, unterschreitet. In diesem Fall gilt der Test als korrekt durchgeführt. Die hierfür benötigte Zeitdauer der Zeitnehmung wird zur Verfügung gehalten und gegebenenfalls angezeigt. Solche Messungenauigkeiten beruhen einerseits auf Ungenauigkeiten des Messgeräts, andererseits auf den notwendigerweise mit dem Gehen oder Stehen verbundenen Bewegungen des Probanden. Beispielsweise kann als tolerierbare Messungenauigkeit ein Schwellenwert von 10 bis 20 cm angenommen werden.

Zur weiteren Validierung bzw. Invalidierung des Tests können noch einzelne Signalparameter im Detail untersucht werden. Überschreitet die Steigung des Signals d(t) einen vorgegebenen Steigungsschwellenwert, so kann davon ausgegangen werden, dass sich der Proband aus dem Aufnahmebereich 60 des Entfernungsmessgeräts 6 bewegt hat, was einen sprunghaften Anstieg des Entfernungsmesswerts 6 bedingt. Generell kann der Steigungsschwellenwert des Signals d(t) mit der höchsten vom Probanden 1 zu erwartenden Geschwindigkeit, gegebenenfalls erhöht um einen Toleranzbereich, angesetzt werden. Für das vorliegende Ausführungsbeispiel kann der Steigungsschwellenwert mit etwa 5m/s festgelegt werden.

Werden im Signal Entfernungsmesswerte d erfasst, die der Entfernung des Stuhls 4 bzw. der Lehne 5 des Stuhls 4 von der Wand 2 entsprechen, so kann davon ausgegangen werden, dass sich der Proband 1 nicht im Aufnahmebereich 60 des Entfernungsmessgeräts 6 befindet, da andernfalls die Erfassung des Probanden 1 im Aufnahmebereich 60 zwischen dem Entfernungsmessgerät 6 und der Wand 2 zu einem geringeren Entfernungsmesswert d führen würde. Aus diesem Grund kann ein Test invalidiert bzw. als nicht korrekt verworfen werden, wenn das Signal d(t) Entfernungsmesswerte d aufweist, die der Entfernung der Lehne 5 des Stuhls 4 von der Wand 2 entsprechen.

Ein weiteres Kriterium für die. Invalidierung eines im Rahmen der Testdurchführung ermittelten Signals d(t) besteht darin, das Signal d(t) darauf zu untersuchen, ob der Proband 1 tatsächlich in einen in den Umkehrbereich 32 vor der Wand 2 gelangt ist, d.h. einen Mindestabstand d_{th,u} von der Sessellehne 5 erreicht hat, der im Ausführungsbeispiel des Timed-Up-and-Go-Tests etwa bei 3m liegt. Erreichen die Entfernungsmesswerte d des Signals d(t) den Umkehrbereich 32 bzw. den Minimalabstand d_{th,u} nicht, und wird der Test vorher beendet, so kann der Test als inkorrekt angesehen und dessen Ergebnis verworfen werden, da davon auszugehen ist, dass der Proband 1 die Wand 2 während der Testdurchführung nicht erreicht hat.

Darüber hinaus kann der Test auch dann abgebrochen werden, wenn die Zeitnehmung eine Maximalzeit erreicht, ohne dass der Test beendet wird. In manchen Fällen können Stürze oder geistige Verwirrung des Probanden dazu führen, dass diese nicht wieder auf dem Stuhl 4 zurückfindet.

Darüber hinaus kann auch während der gesamten Testdurchführung laufend überwacht werden, ob die Ausrichtung des Entfernungsmessgeräts 6 der ursprünglich eingestellten Ausrichtung entspricht oder ob Änderungen der Ausrichtung des Entfernungsmessgeräts 6 zu einer Verfälschung des Timed-Up-and-Go-Tests geführt haben. Bei Änderungen der Ausrichtung des Entfernungsmessgeräts 6 oder bei einem Schwellenwert übersteigenden Abweichungen der Ausrichtung des Entfernungsmessgeräts 6 von einer vorgegebenen, insbesondere horizontalen und/oder normal zur Wand stehenden Ausrichtungen wird eine Fehlermeldung ausgegeben und das Messergebnis wird invalidiert.

Eine weitere Überprüfung am Ende des Tests kann eine weitere Verbesserung der Validität des Tests bringen. Hierfür wird der Proband 1 angewiesen, nach Beendigung des Tests aufzustehen und hinter den Stuhl 4 zu gehen oder den Aufnahmebereich des Entfernungsmessgeräts 6 zu verlassen. Anschließend werden die, bereits zuvor genannten Kriterien zur Beurteilung der korrekten Position und/oder Ausrichtung des Stuhls 4 zur Wand 2 oder zum Hindernis erneut geprüft.

In diesem Zusammenhang kann beispielsweise die Ausrichtung des Stuhls zur Wand 2 erneut überprüft werden: Abweichungen der Ausrichtung des Entfernungsmessgeräts 6 von einer vorgegebenen, insbesondere horizontalen und/oder normal zur Wand 2 oder zum Hindernis stehenden, Ausrichtung führen zur Invalidierung des Messergebnisses.

Ebenso kann auch der ermittelten Entfernungsmesswerte nach dem Abschluss des Tests mit einem vorgegebenen Schwellenwert verglichen werden und derart eine korrekte Position des Stuhls festgestellt werden, wenn der ermittelten Entfernungsmesswert innerhalb eines vorgegebenen Entfernungsbereichs liegt. Ist dies nicht der Fall, wird der Test als inkorrekt angesehen.

## Patentansprüche

1. Verfahren zur Bestimmung der für einen Timed-Up-and-Go-Test benötigten Zeit sowie zur Verifikation des ermittelten Ergebnisses,
- wobei ein Entfernungsmessgerät (6) vorgesehen ist, das den Abstand eines vor einem Stuhl (4) befindlichen Probanden (1) von der Rückenlehne (5) des Stuhls (4) laufend misst, und an eine Steuereinheit (10) weiterleitet,
- wobei der Stuhl (4) positioniert wird, insbesondere zu einer Wand (2) oder einem Hindernis oder einer Markierung in einen vorgegebenen Abstand gebracht wird,
- wobei die vom Entfernungsmessgerät (6) aufgezeichneten Entfernungsmesswerte (d) laufend aufgezeichnet werden,
- wobei der Proband (1) angewiesen wird, vom Stuhl (4) aufzustehen, vorwärts, insbesondere zur Wand (2), zum Hindernis oder zur Markierung zu gehen, anschließend, insbesondere vor der Wand (2) oder dem Hindernis oder im Bereich der Markierung, umzukehren und sich wieder auf dem Stuhl (4) hinzusetzen und der Proband (1) diese Anweisung nach seinen Möglichkeiten ausführt,
- wobei überprüft wird, ob der Proband (1) auf dem Stuhl (4) sitzt und wenn dies für eine vorgegebene Zeitspanne der Fall ist, eine Zeitnehmung gestartet wird und ein vorgegebenes Start-Signal abgegeben wird,
- wobei die Zeitnehmung gestoppt wird, wenn sich der Proband (1) wieder auf den Stuhl (4) setzt,
- wobei aus den über die Zeit aufgenommenen Entfernungsmesswerten ein Signal (d(t)) erstellt wird, das insbesondere nach dem Ende der Zeitnehmung, daraufhin untersucht wird, ob das Signal (d(t))
a) in einem ersten Zeitbereich kontinuierlich ansteigt, gegebenenfalls auch für einzelne Zeiträume bis auf Messungenauigkeiten unverändert bleibt, und
b) nach dem Übersteigen eines vorgegebenen Umkehrschwellenwerts (d_{th,u}) wieder kontinuierlich abnimmt, gegebenenfalls auch für einzelne Zeiträume bis auf Messungenauigkeiten unverändert bleibt, bis es den unteren Schwellenwert (d_{th,s}) unterschreitet, und
- sofern die Vorraussetzungen a) und b) vorliegen, der Test als korrekt durchgeführt angesehen wird und die hierfür benötigte Zeitdauer der Zeitnehmung zur Verfügung gehalten, gegebenenfalls, angezeigt wird.

2. Verfahren nach Anspruch 1, **dadurch gekennzeichnet, dass** vor der Ausführung des Tests die ermittelten Entfernungsmesswerte laufend untersucht werden und für den Fall, dass die Entfernungsmesswerte für eine vorgegebene Zeitspanne einen unteren Schwellenwert unterschreiten, insbesondere wenn der Proband sitzt,
a) das Start-Signal abgegeben wird, das die Zeitnehmung auslöst und dem Probanden signalisiert, dass der Test beginnt, oder
b) die Zeitnehmung gestartet wird, wenn die Entfernungsmesswerte einen Schwellenwert übersteigen, insbesondere wenn der Proband aufsteht

3. Verfahren nach Anspruch 1 oder 2, **dadurch gekennzeichnet,**
- **dass** ein externes Triggersignal die Abgabe des Start-Signals und den Beginn der Zeitnehmung veranlasst, oder dass die Zeitnehmung gestartet wird, wenn ein in der Sitzfläche des Stuhls (4) gelegener Sensor, insbesondere Drucksensor den Abfall des Drucks unter einen vorgegebenen Schwellenwert feststellt,
- **dass**, wenn die Entfernungsmesswerte nach erstmaliger und einen vorgegebenen Zeitraum andauernder Überschreitung eines weiteren Schwellenwerts für einen vorgegebene Zeitspanne einen unteren Schwellenwert unterschreiten, insbesondere wenn der Proband (1) wieder auf dem Stuhl (4) sitzt, der Test beendet und die Zeitnehmung gestoppt wird und
- **dass** ein externes Triggersignal die Zeitnehmung beendet, oder dass die Zeitnehmung beendet wird, wenn ein in der Sitzfläche des Stuhls (4) gelegener Sensor, insbesondere Drucksensor, einen Schwellenwert übersteigenden Druck feststellt.

4. Verfahren nach einem der vorangehenden Ansprüche, **dadurch gekennzeichnet, dass** der Test als inkorrekt angesehen wird,
- wenn die Steigung des Signals (d(t)) einen vorgegebenen Steigungsschwellenwert überschreitet und/oder
- wenn das Signal (d(t)) Messwerte aufweist, die der Entfernung des Stuhls (5) von der Wand (2) oder vom Hindernis entsprechen oder Messwerte aufweist, die die Entfernung des Stuhls zur Markierung um einen vorgegebenen Schwellenwert übersteigen, und/oder
- wenn keiner der Entfernungsmesswerte (d) des Signals (d(t)) einen vorgegebenen Schwellenwert (d_{th,u}) erreicht und der Proband den Umkehrbereich (32) nicht erreicht, und/oder
- wenn die Zeitnehmung eine Maximalzeit erreicht, ohne dass der Test beendet wird.

5. Verfahren nach einem der vorangehenden Ansprüche wobei der Stuhl gegenüber einer Wand (2) oder einem Hindernis positioniert wird, **dadurch gekennzeichnet,**
- **dass** die Ausrichtung des Entfernungsmessgeräts (6) gegenüber der Wand (2) oder dem Hindernis laufend überwacht wird und bei Änderungen der Ausrichtung Entfernungsmessgeräts (6) oder bei einen Schwellenwert übersteigenden Abweichungen der Ausrichtung des Entfernungsmessgeräts (6) von einer vorgegebenen, insbesondere horizontalen und/oder normal zur Wand (2) oder zum Hindernis stehenden, Ausrichtung eine Fehlermeldung ausgegeben und das Messergebnis invalid wird und
- **dass** vor der Ausführung des Tests das Entfernungsmessgerät (6) auf der Lehne (5) des Stuhls (4) angeordnet und von der Lehne (5) in Richtung zum sitzenden Probanden (1) hin ausgerichtet wird.

6. Verfahren nach einem der vorangehenden Ansprüche wobei der Stuhl gegenüber einer Wand (2) oder einem Hindernis positioniert wird, **dadurch gekennzeichnet, dass** die vor dem Beginn des Tests ermittelten Entfernungsmesswerte (d) mit einem vorgegebenen Schwellenwert verglichen werden und derart eine korrekte Position des Stuhls festgestellt wird,
- wenn die ermittelten Entfernungsmesswerte innerhalb eines vorgegebenen Entfernungsbereichs liegen und/oder
- wenn die Ausrichtung des Entfernungsmessgeräts (6) einer vorgegebenen, insbesondere horizontalen und/oder normal zur Wand (2) oder zum Hindernis stehenden Ausrichtung entspricht.

7. Verfahren nach einem der vorangehenden Ansprüche, **dadurch gekennzeichnet, dass** der Proband (1) angewiesen wird, nach Beendigung des Tests aufzustehen und hinter den Stuhl zu gehen oder den Aufnahmebereich des Entfernungsmessgeräts (6) zu verlassen, und dass anschließend die erneut korrekte Position und/oder Ausrichtung des Stuhls (4) erneut nach vorgegebenen Kriterien, insbesondere nach Anspruch 5 oder 6, geprüft wird und der Test als inkorrekt angesehen wird, wenn die Position und/oder Ausrichtung des Stuhls zur Wand (2) nicht diesen Kriterien entspricht.

8. Verfahren nach Anspruch 6, **dadurch gekennzeichnet, dass** festgestellt wird, ob der Abstand des Stuhls (4) bzw. dessen Lehne (5) von der Wand (2) oder vom Hindernis in Bezug auf den Entfernungsbereich zu groß oder zu klein ist und dies entsprechend anzeigt.

9. Verfahren nach einem der vorangehenden Ansprüche, **dadurch gekennzeichnet,**
- **dass** das Entfernungsmessgerät (6) die Entfernung mittels Ultraschall- oder Lasermessung bestimmt und
- **dass** ein Umkehrsignal abgegeben wird, wenn die Entfernungsmesswerte des Entfernungsmessgeräts einen vorgegebenen Abstand überschreiten, insbesondere dann, wenn sich der Proband (1) im Umkehrbereich befindet.

10. Vorrichtung zur Bestimmung der für einen Timed-Up-and-Go-Test benötigten Zeit sowie zur Verifikation des ermittelten Ergebnisses, umfassend
- einen Stuhl (4) mit einer Sitzfläche und einer Rückenlehne (5),
- eine mit dem Stuhl verbundene Steuereinheit (10),
- ein an die Steuereinheit (10) angeschlossenes und mit der Rückenlehne verbundenes Entfernungsmessgerät (6), das den Abstand eines vor einem Stuhl (4) befindlichen Objekts oder Probanden (1) von der Rückenlehne (5) des Stuhls (4) laufend misst, und
- ein der Steuereinheit (10) nachgeschalteter Signalgeber, der ausgebildet ist, bei Vorliegen eines Trigger-Signals oder bei Feststellung, dass ein Proband auf dem Stuhl sitzt, ein Startsignal abzugeben,
- eine Zeitgebung, die durch das Startsignal aktivierbar ist,
wobei die Steuereinheit (10) dazu ausgebildet ist, aus den ihr zugehenden Entfernungsmesswerten ein Signal (d(t)) erstellt, und dieses, insbesondere nach dem Ende der Zeitnehmung, daraufhin untersucht, ob das Signal
a) in einem ersten Zeitbereich kontinuierlich ansteigt, gegebenenfalls auch für einzelne Zeiträume bis auf Messungenauigkeiten unverändert bleibt, und
b) nach dem Übersteigen eines vorgegebenen Umkehrschwellenwerts (d_{th,u}) wieder kontinuierlich abnimmt, gegebenenfalls auch für einzelne Zeiträume bis auf Messungenauigkeiten unverändert bleibt, bis es den unteren Schwellenwert (d_{th,s}) unterschreitet, und
wobei die Steuereinheit (10) dazu ausgebildet ist, die Validität des Tests festzustellen und gegebenenfalls die von der Zeitnehmung ermittelte Zeit anzuzeigen oder zur Verfügung zu halten, wenn die Vorraussetzungen a) und b) vorliegen.

11. Vorrichtung nach Anspruch 10, **dadurch gekennzeichnet, dass** die Steuereinheit (10) dazu ausgebildet ist, vor der Ausführung des Tests die ermittelten Entfernungsmesswerte laufend zu untersuchen und für den Fall, dass die Entfernungsmesswerte für eine vorgegebene Zeitspanne einen unteren Schwellenwert unterschreiten, insbesondere wenn der Proband sitzt,
a) ein Start-Signal abzugeben, die Zeitnehmung auszulösen und somit dem Probanden zu signalisieren, dass der Test beginnt, oder
b) die Zeitnehmung zu starten, wenn die Entfernungsmesswerte einen Schwellenwert übersteigen, insbesondere wenn der Proband aufsteht.

12. Vorrichtung nach Anspruch 10 oder 11, **dadurch gekennzeichnet,**
- **dass** die Steuereinheit (10) einen Steuereingang für ein externes Triggersignal aufweist, und dass die Steuereinheit (10) dazu ausgebildet ist, bei Vorliegen eines solchen externen Triggersignals das Start-Signal abzugeben und die Zeitnehmung zu starten, oder dass der Stuhl (4) einen Drucksensor im Bereich der Sitzfläche aufweist, der an die Steuereinheit (10) angeschlossen ist und dass die Steuereinheit (10) dazu ausgebildet ist, die Zeitnehmung zu aktivieren, wenn der Sensor den Abfall des Drucks unter einen vorgegebenen Schwellenwert feststellt und/oder
- **dass** die Steuereinheit (10) dazu ausgebildet ist, für den Fall, dass die Entfernungsmesswerte nach erstmaliger und einen vorgegebenen Zeitraum andauernder Überschreitung eines weiteren Schwellenwerts für einen vorgegebene Zeitspanne einen unteren Schwellenwert unterschreiten, insbesondere wenn der Proband (1) wieder auf dem Stuhl (4) sitzt, den Test zu beenden und die Zeitnehmung zu stoppen und/oder
- **dass** die Steuereinheit (10) einen Steuereingang für ein externes Triggersignal aufweist, und dass die Steuereinheit (10) dazu ausgebildet ist, bei Vorliegen eines externen Triggersignals die Zeitnehmung zu beenden, oder dass der Stuhl (4) einen Drucksensor im Bereich der Sitzfläche aufweist, der an die Steuereinheit (10) angeschlossen ist und dass die Steuereinheit (10) dazu ausgebildet ist,
die Zeitnehmung zu beenden, wenn der Drucksensor, einen Anstieg des Drucks über einen vorgegebenen Schwellenwert feststellt.

13. Vorrichtung nach einem der Ansprüche 10 bis 12, **dadurch gekennzeichnet, dass** die Steuereinheit (10) dazu ausgebildet ist, den Test zu invalidieren,
- wenn die Steigung des Signals (d(t)) einen vorgegebenen Steigungsschwellenwert überschreitet und/oder
- wenn das Signal (d(t)) Messwerte aufweist, die der Entfernung des Stuhls (5) von der Wand (2) oder vom Hindernis entsprechen oder Messwerte aufweist, die die Entfernung des Stuhls zur Markierung um einen vorgegebenen Schwellenwert übersteigen, und/oder
- wenn keiner der Entfernungsmesswerte (d) des Signals (d(t)) einen vorgegebenen Schwellenwert (d_{th,u}) erreicht und der Proband den Umkehrbereich (32) nicht erreicht, und/oder
- wenn die Zeitnehmung eine Maximalzeit erreicht, ohne dass der Test beendet wurde.

14. Vorrichtung nach einem der Ansprüche 10 bis 13, **dadurch gekennzeichnet, dass** das Entfernungsmessgerät (6) auf der Lehne (5) des Stuhls (4) angeordnet ist und von der Lehne (5) in Richtung zum sitzenden Probanden (1) hin ausgerichtet ist und/oder
- dass das Entfernungsmessgerät (6) die Entfernung mittels Ultraschall- oder Lasermessung bestimmt.

## Claims

1. Method for determining the time required for a Timed Up And Go test and for verifying the result obtained,
- wherein a distance measuring device (6) is provided which continuously measures and transmits to a control unit (10) the distance of a subject (1) located in front of a chair (4) from the backrest (5) of the chair (4),
- wherein the chair (4) is positioned, in particular is brought to a predetermined distance from a wall (2) or an obstacle or a mark,
- wherein the distance measurement values (d) recorded by the distance measuring device (6) are continuously recorded,
- wherein the subject (1) is instructed to stand up from the chair (4), to move forward, in particular towards the wall (2), the obstacle or the mark, then to turn around, in particular in front of the wall (2) or the obstacle or in the area of the mark, and to again sit on the chair (4), and the subject (1) carries out this instruction according to their capabilities,
- wherein it is checked whether the subject (1) is seated on the chair (4) and, if this is the case for a predetermined timespan, a time recordal is started and a predetermined start signal is given,
- wherein the time recordal is stopped when the subject (1) again sits on the chair (4),
- wherein a signal (d(t)) is generated from the distance measurement values recorded over time, which signal (d(t)) is examined, in particular after the end of the time recordal, to determine whether the signal (d(t))
(a) increases continuously in a first time range, or, as appropriate, remains for individual time intervals unchanged apart from measurement errors, and
(b) continuously decreases again after exceeding a predetermined turn-around threshold (d_{th,u}) or, as appropriate, remains for individual time intervals unchanged apart from measurement errors, until it falls below the lower threshold (d_{th,s}), and
- provided that conditions (a) and (b) are met, the test is considered to have been carried out correctly and the length of time required is kept available or, as appropriate, displayed.

2. Method according to claim 1, **characterised in that** before the test is carried out, the determined distance measurements are continuously examined and, in the event that the distance measurements fall below a lower threshold value for a predetermined timespan, in particular if the subject is seated,
(a) the start signal is given, which initiates the time recordal and signals to the subject that the test is beginning; or
(b) the time recordal is started when the distance readings exceed a threshold value, in particular when the subject stands up.

3. Process according to claim 1 or 2, **characterised in that**
- an external trigger signal causes the start signal to be emitted and the time recordal to begin, or the time recordal is started when a sensor located in the seat area of the chair (4), in particular a pressure sensor, detects the drop in pressure below a predetermined threshold value,
- if the distance measurement values fall below a lower threshold value for a predetermined time interval after a further threshold value has been exceeded for the first time and continues to be exceeded for a predetermined timespan, in particular if the subject (1) again sits on the chair (4), the test is terminated and the time recordal is stopped, and
- an external trigger signal terminates the time recordal, or the time recordal is terminated when a sensor, in particular a pressure sensor, located in the seat area of the chair (4) detects a pressure exceeding a threshold value.

4. Method according to any one of the preceding claims, **characterised in that** the test is considered incorrect,
- if the gradient of the signal (d(t)) exceeds a predetermined gradient threshold and/or
- if the signal (d(t)) has measured values which correspond to the distance of the chair (5) from the wall (2) or obstacle or has measured values which exceed the distance of the chair from the mark by a predetermined threshold value, and/or
- if none of the distance measurement values (d) of the signal (d(t)) reaches a predetermined threshold value (d_{th,u}) and the subject does not reach the turn-around area (32), and/or
- if the time recordal reaches a maximum time without the test being terminated.

5. Method according to any one of the preceding claims wherein the chair is positioned relative to a wall (2) or an obstacle, **characterised in that**
- the orientation of the distance measuring device (6) relative to the wall (2) or the obstacle is continuously monitored and, in the event of changes in the orientation of the distance measuring device (6) or in the event of deviations in the orientation of the distance measuring device (6) from a predetermined orientation, in particular a horizontal orientation and/or an orientation normal to the wall (2) or the obstacle, that exceed a threshold value, an error message is output and the measurement result becomes invalid and
- before the test is carried out, the distance measuring device (6) is arranged on the backrest (5) of the chair (4) and is oriented from the backrest (5) in the direction of the seated subject (1).

6. Method according to any one of the preceding claims wherein the chair is positioned relative to a wall (2) or an obstacle, **characterised in that** the distance measurement values (d) determined before the start of the test are compared with a predetermined threshold value and **in that** way a correct position of the chair is determined,
- if the determined distance measurement values lie within a predetermined distance range and/or
- if the orientation of the distance measuring device (6) corresponds to a predetermined orientation, in particular horizontal and/or normal to the wall (2) or obstacle.

7. Method according to any one of the preceding claims, **characterised in that** the test subject (1) is instructed to stand up after termination of the test and to walk behind the chair or leave the recording area of the distance measuring device (6), and that subsequently the again correct position and/or orientation of the chair (4) is again checked according to predetermined criteria, in particular according to claim 5 or 6, and the test is regarded as incorrect if the position and/or orientation of the chair relative to the wall (2) does not meet those criteria.

8. Method according to claim 6, **characterised in that** it is determined whether the distance of the chair (4) or its backrest (5) from the wall (2) or from the obstacle is too large or too small in relation to the distance range and this displays accordingly.

9. Method according to any one of the preceding claims, **characterised in that**
- the distance measuring device (6) determines the distance by means of ultrasonic or laser measurement, and
- a turn-around signal is emitted when the distance measurement values of the distance measuring device exceed a predetermined distance, in particular when the subject (1) is in the turn-around area.

10. Device for determining the time required for a Timed Up And Go test and for verifying the result obtained, comprising
- a chair (4) with a seat and a backrest (5),
- a control unit (10) connected to the chair,
- a distance measuring device (6) connected to the control unit (10) and to the backrest, which continuously measures the distance of an object or subject (1) located in front of a chair (4) from the backrest (5) of the chair (4), and
- a signal generator downstream of the control unit (10), which is configured to emit a start signal when a trigger signal is present or when it is established that a subject is seated on the chair,
- a timing that can be activated by the start signal,
wherein the control unit (10) is configured to generate a signal (d(t)) from the distance measurement values it receives, and to examine this signal, in particular after the end of the time recordal, to determine whether the signal
(a) increases continuously in a first time range, or, as appropriate, remains for individual time intervals unchanged apart from measurement errors, and
(b) continuously decreases again after exceeding a predetermined turn-around threshold (d_{th,u}) or, as appropriate, remains for individual time intervals unchanged apart from measurement errors, until it falls below the lower threshold (d_{th,s}), and
wherein the control unit (10) is configured to determine the validity of the test and, as appropriate, to display or keep available the time determined by the time recordal provided conditions a) and b) are met.

11. Device according to claim 10, **characterised in that** the control unit (10) is configured to continuously examine the determined distance measurement values before the test is carried out and in the event that the distance measurement values fall below a lower threshold value for a predetermined timespan, in particular if the subject is seated,
(a) to emit a start signal, to initiate the time recordal and thus to signal to the subject that the test is beginning; or
(b) to start the time recordal if the distance measurement values exceed a threshold value, in particular if the subject stands up.

12. Device according to claim 10 or 11, **characterised in that**
- the control unit (10) has a control input for an external trigger signal, and **in that** the control unit (10) is configured to emit the start signal and to start the time recordal when such an external trigger signal is present, or the chair (4) has a pressure sensor in the region of the seat area which is connected to the control unit (10), and the control unit (10) is configured to activate the time recordal when the sensor detects the drop in pressure below a predetermined threshold value and/or
- the control unit (10) is configured, in the event that distance measurement values fall below a lower threshold value for a predetermined timespan after a further threshold value has been exceeded for the first time and continues to be exceeded for a predetermined time interval, in particular if the subject (1) again sits on the chair (4), to terminate the test and to stop the time recordal and/or
- the control unit (10) has a control input for an external trigger signal, and the control unit (10) is configured to terminate the time recordal if an external trigger signal is present, or the chair (4) has a pressure sensor in the region of the seat area which is connected to the control unit (10), and the control unit (10) is configured to terminate the time recordal if the pressure sensor detects an increase in pressure above a predetermined threshold value.

13. Device according to any one of the claims 10 to 12, **characterised in that** the control unit is configured to invalidate the test
- if the gradient of the signal (d(t)) exceeds a predetermined gradient threshold and/or
- if the signal (d(t)) has measured values which correspond to the distance of the chair (5) from the wall (2) or obstacle or has measured values which exceed the distance of the chair from the mark by a predetermined threshold value, and/or
- if none of the distance measurement values (d) of the signal (d(t)) reaches a predetermined threshold value (d_{th,u}) and the subject does not reach the turn-around area (32), and/or
- if the time recordal reaches a maximum time without the test having been terminated.

14. Device according to any one of the claims 10 to 13, **characterised in that** the distance measuring device (6) is arranged on the backrest (5) of the chair (4) and is oriented from the backrest (5) in the direction of the seated subject (1) and/or
- the distance measuring device (6) determines the distance by means of ultrasonic or laser measurement.

## Revendications

1. Procédé permettant de déterminer le temps nécessaire pour un test de lever de chaise chronométré ainsi que permettant de vérifier le résultat obtenu,
- dans lequel un appareil de mesure de distance (6) est prévu, qui mesure en continu la distance entre un sujet (1) se trouvant devant une chaise (4) et le dossier (5) de ladite chaise (4) et qui la transmet à une unité de commande (10),
- dans lequel la chaise (4) est positionnée, en particulier est placée à une distance prédéfinie d'un mur (2) ou d'un obstacle ou d'un repère,
- dans lequel les valeurs de mesure de distance (d) enregistrées par l'appareil de mesure de distance (6) sont enregistrées en continu,
- dans lequel il est demandé au sujet (1) de se lever de la chaise (4), de se diriger vers l'avant, en particulier vers le mur (2), vers l'obstacle ou vers le repère, puis de faire demi-tour, en particulier devant le mur (2) ou l'obstacle ou dans la zone du repère et de revenir s'asseoir sur la chaise (4), et le sujet (1) met en oeuvre cette demande en fonction de ses possibilités,
- dans lequel il est vérifié que le sujet (1) est assis sur la chaise (4) et, si c'est le cas pendant un laps de temps prédéfini, un chronométrage est démarré et un signal de départ prédéfini est émis,
- dans lequel le chronométrage est arrêté lorsque le sujet (1) se rassoit sur la chaise (4),
- dans lequel un signal (d(t)) est créé à partir des valeurs de mesure de distance enregistrées sur la période, ledit signal (d(t)) étant ensuite examiné, en particulier après la fin du chronométrage, afin de savoir s'il
a) augmente de manière continue dans un premier domaine temporel, et aussi s'il demeure éventuellement inchangé pour des périodes individuelles, exception faite des imprécisions de mesure, et
b) décroit à nouveau de manière continue après le dépassement d'une valeur de seuil de demi-tour (d_{th,u}), et aussi s'il demeure éventuellement inchangé pour des périodes individuelles, exception faite des imprécisions de mesure, jusqu'à ce qu'il descende en dessous de la valeur de seuil (d_{th,s}) inférieure, et
- dans la mesure où les conditions préalables a) et b) sont remplies, le test est considéré comme ayant été mis en oeuvre correctement et la durée nécessaire du chronométrage pour ledit test est mise à disposition et éventuellement indiquée.

2. Procédé selon la revendication 1, **caractérisé en ce que** les valeurs de mesure de distance déterminées sont examinées en continu avant la mise en œuvre du test et, dans le cas où les valeurs de mesure de distance descendent en dessous d'une valeur de seuil inférieure pendant un laps de temps prédéfini, en particulier lorsque le sujet est assis,
a) le signal de départ est émis, ce qui déclenche le chronométrage et signale au sujet que le test commence, ou
b) le chronométrage est démarré lorsque les valeurs de mesure de distance dépassent une valeur de seuil, en particulier lorsque le sujet se lève.

3. Procédé selon la revendication 1 ou 2, **caractérisé en ce que,**
- un signal de déclenchement externe déclenche l'émission du signal de départ et le début du chronométrage, ou **en ce que** le chronométrage est démarré lorsqu'un capteur, en particulier un capteur de pression, placé dans l'assise de la chaise (4) constate la chute de la pression en dessous d'une valeur de seuil prédéfinie,
- si les valeurs de mesure de distance descendent en dessous d'une valeur de seuil inférieure pendant un laps de temps prédéfini après qu'une autre valeur de seuil a été dépassée pour la première fois et de manière persistante, en particulier si le sujet (1) se rassoit sur la chaise (4), le test est achevé et le chronométrage est arrêté et
- un signal de déclenchement externe achève le chronométrage, ou **en ce que** le chronométrage est achevé lorsqu'un capteur, en particulier un capteur de pression, placé dans l'assise de la chaise (4) constate une pression dépassant une valeur de seuil.

4. Procédé selon l'une quelconque des revendications précédentes, **caractérisé en ce que** le test est considéré comme incorrect,
- si la pente du signal (d(t)) dépasse une valeur de seuil de pente prédéfinie et/ou
- si le signal (d(t)) présente des valeurs de mesure qui correspondent à la distance entre la chaise (5) et le mur (2) ou l'obstacle ou présente des valeurs de mesure qui dépassent d'une valeur de seuil prédéfinie la distance entre la chaise et le repère, et/ou
- si aucune des valeurs de mesure de distance (d) du signal (d(t)) n'atteint une valeur de seuil (d_{th,u}) prédéfinie et le sujet n'atteint pas la zone de demi-tour (32), et/ou
- si le chronométrage atteint un temps maximal sans que le test n'ait été achevé.

5. Procédé selon l'une quelconque des revendications précédentes, dans lequel la chaise est positionnée en face d'un mur (2) ou d'un obstacle, **caractérisé en ce que**,
- l'orientation de l'appareil de mesure de distance (6) face au mur (2) ou à l'obstacle est surveillée en continu et en cas de modification de l'orientation de l'appareil de mesure de distance (6) ou en cas de déviations de l'orientation de l'appareil de mesure de distance (6) par rapport à une orientation prédéfinie, en particulier horizontale et/ou perpendiculaire au mur (2) ou à l'obstacle, qui dépasseraient une valeur de seuil, un message d'erreur est émis et le résultat de la mesure est invalidé et
- avant la mise en œuvre du test, l'appareil de mesure de distance (6) est agencé sur le dossier (5) de la chaise (4) et orienté depuis le dossier (5) en direction du sujet (1) assis.

6. Procédé selon l'une quelconque des revendications précédentes, dans lequel la chaise est positionnée face à un mur (2) ou un obstacle, **caractérisé en ce que** les valeurs de mesure de distance(d) déterminées avant le début du test sont comparées à une valeur de seuil prédéfinie et une position correcte de la chaise est de cette manière constatée,
- si les valeurs de mesure de distance déterminées se situent dans une plage de distance prédéfinie et/ou
- si l'orientation de l'appareil de mesure de distance (6) correspond à une orientation prédéfinie, en particulier horizontale et/ou perpendiculaire au mur (2) ou à l'obstacle.

7. Procédé selon l'une quelconque des revendications précédentes, **caractérisé en ce qu'**il est demandé au sujet (1) de se lever après l'achèvement du test et de passer derrière la chaise ou de quitter la zone d'enregistrement de l'appareil de mesure de distance (6), et **en ce que** la position et/ou l'orientation de la chaise (4) à nouveau correcte est ensuite à nouveau vérifiée selon des critères prédéfinis, en particulier selon la revendication 5 ou 6, et le test est considéré comme incorrect si la position et/ou l'orientation de la chaise par rapport au mur (2) ne correspond pas auxdits critères.

8. Procédé selon la revendication 6, **caractérisé en ce qu'**il est constaté que la distance entre la chaise (4) ou son dossier (5)et le mur (2) ou l'obstacle est trop grande ou trop petite par rapport à la plage de distance et cela indique de manière correspondante.

9. Procédé selon l'une quelconque des revendications précédentes, **caractérisé en ce que**,
- l'appareil de mesure de distance (6) détermine la distance au moyen d'une mesure par ultrasons ou laser et
- un signal de demi-tour est émis lorsque les valeurs de mesure de distance de l'appareil de mesure de distance dépassent une distance prédéfinie, en particulier lorsque le sujet (1) se trouve dans la zone de demi-tour.

10. Procédé permettant de déterminer le temps nécessaire pour un test de lever de chaise chronométré ainsi que permettant de vérifier le résultat obtenu, comprenant
- une chaise (4) avec une assise et un dossier (5),
- une unité de commande (10) reliée à la chaise,
- un appareil de mesure de distance (6), connecté à l'unité de commande (10) et relié au dossier, qui mesure en continu la distance entre un objet ou un sujet (1) se trouvant devant une chaise (4) et le dossier (5) de la chaise (4), et
- un émetteur de signaux connecté en aval de l'unité de commande (10) et conçu pour émettre un signal de départ lorsque se présente un signal de déclenchement ou lorsqu'il est constaté qu'un sujet est assis sur la chaise,
- une temporisation pouvant être activée grâce au signal de départ,
dans lequel l'unité de commande (10) est conçue pour générer un signal (d(t)) à partir des valeurs de mesure de distance qui lui parviennent, et examiner ledit signal, en particulier après la fin du chronométrage, pour savoir s'il
a) augmente de manière continue dans un premier domaine temporel, et aussi s'il demeure éventuellement inchangé pour des périodes individuelles, exception faite des imprécisions de mesure, et
b) décroit à nouveau de manière continue après le dépassement d'une valeur de seuil de demi-tour (d_{th,u}), et aussi s'il demeure éventuellement inchangé pour des périodes individuelles, exception faite des imprécisions de mesure, jusqu'à ce qu'il descende en dessous de la valeur de seuil (d_{th,s}) inférieure, et
dans lequel, si les conditions préalables a) et b) sont remplies, l'unité de commande (10) est conçue pour constater la validité du test et éventuellement indiquer ou mettre à disposition le temps déterminé par le chronométrage, si les conditions préalables a) et b) sont satisfaites.

11. Dispositif selon la revendication 10, **caractérisé en ce que** l'unité de commande (10) est conçue pour examiner en continu les valeurs de mesure de distance déterminées avant la mise en œuvre du test et dans le cas où les valeurs de mesure de distance descendent en dessous d'une valeur de seuil inférieure pendant un laps de temps prédéfini, en particulier lorsque le sujet est assis,
a) émettre un signal de départ, déclencher le chronométrage et signaler ainsi au sujet que le test commence, ou
b) démarrer le chronométrage lorsque les valeurs de mesure de distance dépassent une valeur de seuil, en particulier lorsque le sujet se lève.

12. Dispositif selon la revendication 10 ou 11, **caractérisé en ce que**
- l'unité de commande (10) présente une entrée de commande pour un signal de déclenchement externe, et **en ce que** l'unité de commande (10) est conçue pour émettre le signal de départ et démarrer le chronométrage lorsque se présente un tel signal de déclenchement externe, ou **en ce que** la chaise (4) présente dans la région de l'assise un capteur de pression connecté à l'unité de commande (10) et **en ce que** l'unité de commande (10) est conçue pour activer le chronométrage lorsque le capteur constate la chute de la pression en dessous d'une valeur de seuil prédéfinie et/ou
- l'unité de commande (10) est conçue pour achever le test et arrêter le chronométrage dans le cas où les valeurs de mesure de distance descendent en dessous d'une valeur de seuil inférieure pendant un laps de temps prédéfini après qu'une autre valeur de seuil a été dépassée pour la première fois et de manière persistante, en particulier si le sujet (1) se rassoit sur la chaise (4), et/ou
- l'unité de commande (10) présente une entrée de commande pour un signal de déclenchement externe, et **en ce que** l'unité de commande (10) est conçue pour achever le chronométrage lorsque se présente un signal de déclenchement externe, ou **en ce que** la chaise (4) présente dans la région de l'assise un capteur de pression connecté à l'unité de commande (10) et **en ce que** l'unité de commande (10) est conçue pour achever le chronométrage lorsque le capteur de pression constate une augmentation de la pression au-delà d'une valeur de seuil prédéfinie.

13. Dispositif selon l'une quelconque des revendications 10 à 12, **caractérisé en ce que** l'unité de commande (10) est conçue pour invalider le test,
- si la pente du signal (d(t)) dépasse une valeur de seuil de pente prédéfinie et/ou
- si le signal (d(t)) présente des valeurs de mesure qui correspondent à la distance entre la chaise (5) et le mur (2) ou l'obstacle ou présente des valeurs de mesure qui dépassent d'une valeur de seuil prédéfinie la distance entre la chaise et le repère, et/ou
- si aucune des valeurs de mesure de distance (d) du signal (d(t)) n'atteint une valeur de seuil (d_{th,u}) prédéfinie et le sujet n'atteint pas la zone de demi-tour (32), et/ou
- si le chronométrage atteint un temps maximal sans que le test n'ait été achevé.

14. Dispositif selon l'une quelconque des revendications 10 à 13, **caractérisé en ce que** l'appareil de mesure de distance (6) est agencé sur le dossier (5) de la chaise (4) et est orienté depuis le dossier (5) en direction du sujet (1) assis et/ou
- l'appareil de mesure de distance (6) détermine la distance au moyen d'une mesure par ultrasons ou laser.
